# EUROPEAN PATENT APPLICATION

(11) **EP 1 804 083 A2**
(43) Date of publication of application: **04.07.2007**
(21) Application number: 06025989.2
(22) Date of filing: 14.12.2006
(51) Int. Cl.: G01T 1/29

(54) **Nuclear medicine diagnostic apparatus**

(30) Priority: 28.12.2005 JP 2005379408
(71) Applicant: Hitachi, Ltd., Tokyo 100-8280 (JP)
(72) Inventor: Yanagita, Norihito c/o Hitachi Ltd. I.Prop. Office, Chiyoda-ku,Tokyo 100-8220 (JP); Kimura, Shigeru c/o Hitachi Ltd. Int.Prop. Office, Chiyoda-ku,Tokyo 100-8220 (JP); Takakusa, Yasuo c/o Hitachi Ltd. Int. Prop. Office, Chiyoda-ku,Tokyo 100-8220 (JP); Tsuchiya, Katsutoshi c/o Hitachi Ltd. I.P. Office, Chiyoda-ku,Tokyo 100-8220 (JP)
(74) Representative: Strehl Schübel-Hopf & Partner

(57) **Abstract**

A nuclear medicine diagnostic apparatus provided with a correction radiation source is shown.

The nuclear medicine diagnostic apparatus comprises a radiation detector (21) that detects radiation radiated from a test object (D), a correction radiation source (30) used to correct decay generated when the radiation penetrates through the test object (D), a radiation source holding unit (31) that sets an advancing direction of radiation (T) for correction to a predetermined direction, a support device (40) that displaces the radiation source holding unit (31) along a body axis (z) and supports the radiation source holding unit in a manner to enable switching the advancing direction of the radiation (T) for correction, and a revolving device (50) that revolves the support device (40) about the body axis (Z) of the test object (D).

## Description

### Technical Field

The present invention relates to a nuclear medicine diagnostic apparatus making use of radiation, and more particular, to a nuclear medicine diagnostic apparatus provided with a correction radiation source, by which medical diagnosis is made with high accuracy.

### Related Art

Among nuclear medicine diagnostic apparatuses, which make use of radiation, a positron emission computed tomography (referred below to as PET) and a single photon emission computed tomography (referred below to as SPECT) provide for a procedure of giving a radioactive medicine to a test object (patient) to measure the distribution of the given radioactive medicine in a body to obtain a tomographic image. By adopting such procedure, detection of function and metabolism in molecular level can be made and a tomographic image based on a body function can be provided.

As a radioactive medicine as given, PET uses one including a positron emission nuclide (¹⁸F, ¹⁵O, ¹¹C, etc.) and SPECT uses one including a single photon emission nuclide (⁹⁹Tc, ⁶⁷Ga, ²⁰¹T1, etc.).

Here, in explaining PET as a typical example, a radioactive medicine (for example, fluorodeoxyglucose (2-[F-18]fluoro-2-deoxy-D-glucose, FDG) including a position emission nuclide taken into a body of a test object possesses a property of accumulating in a tumor tissue of a test object due to carbohydrate metabolism.

When the positron emission nuclide decomposes in a position of accumulation, it emits positrons (β+), the positrons combining with neighboring electrons to annihilate. When positrons annihilate in this manner, a pair of γ-rays (radiation), respectively, having 511 keV in energy are emitted in mutually opposite directions (180 degrees ± 0.6 degrees).

The pair of γ-rays thus emitted are detected at the same time in two ones of a multiplicity of radiation detectors arranged around a test object. Data thus detected are accumulated to identify and image a position of radiation (that is, a position, in which a radioactive medicine is accumulated), thus enabling specifying a tumor site in a test object.

By the way, with such nuclear medicine diagnostic apparatus, radiation emitted from a nuclide (a position emission nuclide or a single photon emission nuclide) taken into a body of a test object is absorbed or scattered to decay in the course of advancing in the body of the test object. Such decay of radiation leads to a decrease in image quality, of a tomographic image of the test object.

Therefore, a correcting process is provided separately of an imaging process of a tomographic image in order to correct a decrease in image quality. Such correcting process is one, in which a correction radiation source is revolved around a body axis of a test object to permit radiation for correction to be radiated on the test object to obtain correction data, by which decay of radiation and sensitivity of an associated apparatus are corrected (see, for example, JP-A-8-338874 (Paragraphs 0002 to 0003, Figs. 1 and 6).

By the way, there is caused a problem that when a nuclear medicine diagnostic apparatus goes through an imaging process, radiation for correction leaking from a correction radiation source having been withdrawn is erroneously counted (contamination) by a radiation detector whereby a tomographic image is decreased in image quality. When it is tried to withdraw a correction radiation source to a position fairly distant from a radiation detector to accommodate the same in a package, which serves also as shielding of radiation, with a view to avoiding such contamination, there is caused a problem that making a nuclear medicine diagnostic apparatus large-scale is unavoidable.

### Summary of the Invention

The invention has been thought of with a view to solving such problem and has its object to provide a nuclear medicine diagnostic apparatus, which eliminates making the apparatus large-scale and prevents contamination by leakage of radiation for correction in a simple and stable operation to improve a tomographic image of a test object being imaged, in image quality.

In order to solve the problem, the invention provides an nuclear medicine diagnostic apparatus comprising a radiation detector that detects radiation radiated from a test object, a correction radiation source used to correct decay generated when the radiation radiated by the radiation detector penetrates through the test object, a radiation source holding unit that holds the correction radiation source to set an advancing direction of radiation for correction as output to a predetermined direction, support means that displaces the radiation source holding unit along a body axis of the test object and supports the radiation source holding unit in a manner to enable switching the advancing direction of the radiation for correction, and revolving means that revolves the support means about the body axis.

The invention is constructed in this manner whereby the correction radiation source is revolved around the body axis of the test object and radiation for correction is irradiated, thus enabling executing the correcting process. In the case where the correcting process is terminated and shifted to the imaging process, the correction radiation source is withdrawn so as to separate from the test object and the radiation detector and the advancing direction of the radiation for correction is switched, so that the radiation for correction is not radiated on the test object and the radiation detector.

The invention produces the following effect. That is, a nuclear medicine diagnostic apparatus is provided, which eliminates making the apparatus large-scale and prevents contamination by leakage of radiation for correction in a simple and stable operation to improve a tomographic image of a test object being imaged, in image quality.

Other objects, features and advantages of the invention will become apparent from the following description of the embodiments of the invention taken in conjunction with the accompanying drawings.

### Brief Description of the Drawings

Fig. 1 is a perspective view showing a whole construction of a PET apparatus representative of an embodiment of a nuclear medicine diagnostic apparatus according to the invention;
Fig. 2 is a conceptual view schematically showing a circumferential section of an imaging unit in the PET apparatus shown in Fig. 1;
Fig. 3 is a cross sectional view showing a state, in which a test object goes through a correcting process of medical diagnosis, which makes use of radiation, in the PET apparatus shown in Fig. 1;
Fig. 4 is a cross sectional view showing a first embodiment in a state, in which a test object goes through an imaging of medical diagnosis, which makes use of radiation, in the PET apparatus shown in Fig. 1; and
Fig. 5 is a cross sectional view showing a second embodiment in a state, in which a test object goes through an imaging process of medical diagnosis, which makes use of radiation, in the PET apparatus shown in Fig. 1.

### Description of the Preferred Embodiments

### (First embodiment)

A PET apparatus, according to a first embodiment, preferred as a nuclear medicine diagnostic apparatus of the invention will be described in detail appropriately referring to the drawings.

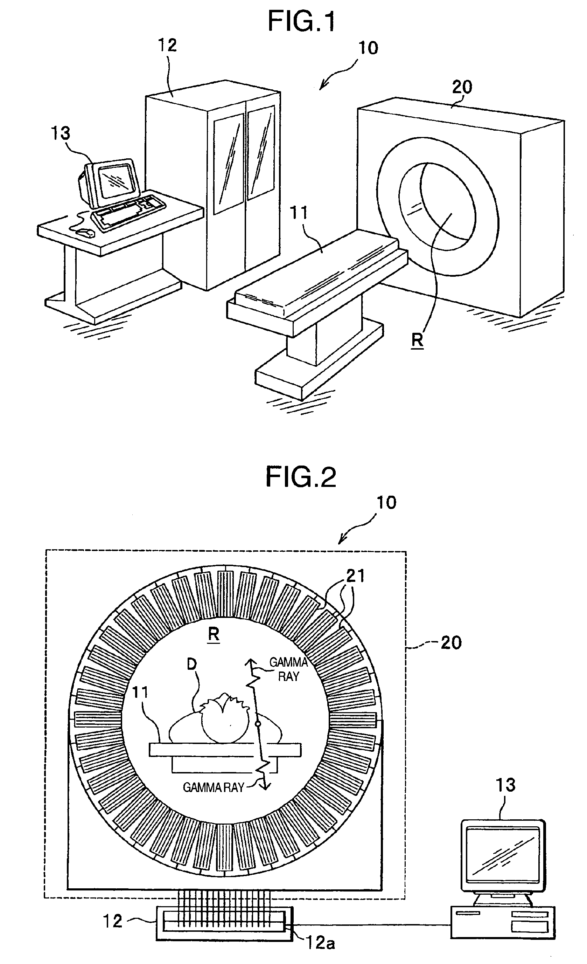

The PET apparatus 10 according to the embodiment comprises, as shown in Fig. 1, a bed 11, a data processing unit 12, a display unit 13, and an imaging unit 20.

With the PET apparatus 10 constructed in this manner, a test object loaded on the bed 11 is inserted into a measurement space R, γ-rays (radiation) radiated from a tumor tissue are detected by the imaging unit 20, the data processing unit 12 subjects the detected signal to data processing to specify points in a coordinate space, from which radiation is generated, and the display unit 13 images an aggregate of the points to display the same as a tomographic image. In this manner, the PET apparatus 10 specifies a tumor site in a body of the test object.

By the way, in the case where the PET apparatus 10 is used to perform medical diagnosis, it is necessary to go through an imaging process, in which a tomographic image is obtained to specify a tumor site, and a correcting process, in which correction data required for an improvement in image quality, of the tomographic image are obtained. The imaging process and the correcting process are executed on the test object loaded on the bed 11 in a state of being inserted into the measurement space R.

An explanation will be continued with reference to Fig. 2.

The bed 11 loads thereon the test object D and fixes thereto the test object D so that a central axis of the measurement space R, which radiation detector units 21 are arranged in a cylindrical-shaped manner to define, and a body axis of the test object D agree with each other.

The test object D has been given FDG, which contains fluorine-18 (¹⁸F) having a half-life period of 110 minutes, positive electrons are emitted from fluorine-18 (¹⁸F) contained in FDG, which is accumulated in a tumor tissue of the test object D due to carbohydrate metabolism, and γ-rays (radiation) generated when the emitted positive electrons and neighboring electrons annihilate in pair are radiated.

The data processing unit 12 can accumulate, every radiation detector, crest values of γ-rays (radiation) detected by a multiplicity of radiation detectors (not shown), which are held by the radiation detector units 21, 21, ... to be arranged densely on an outer peripheral surface of the measurement space R, and data of detection time.

In the correcting process, the data processing unit 12 accumulates, as correction data, crest values of radiation T for correction, having penetrated through the test object D to be detected by the respective radiation detectors. Crest values of the radiation T for correction, obtained in the correcting process include information related to the decay characteristic of radiation when penetrating through the test object D.

Also, in the imaging process, the data processing unit 12 uses an internal simultaneous measuring device 12a to detect, at predetermined time intervals, γ-rays (radiation), which are radiated from within a body of the test object D to be detected by the respective radiation detectors. Positions of those radiation detectors in two locations, which are measured at the same time, are specified by subjecting imaging data obtained in the imaging process to a predetermined correction arithmetic processing on the basis of the correction data. In this manner, points in the coordinate space, from which γ-rays of 511 keV are generated, are specified and accumulated from those positions of plural pairs of radiation detectors, which are specified by the data processing unit 12.

The display unit 13 forms points in the coordinate space, which are specified by the data processing unit 12 and from which γ-rays of 511 keV are generated, into a tomographic image to specify a tumor site of the test object D and carries out various operations for a predetermined medical diagnosis.

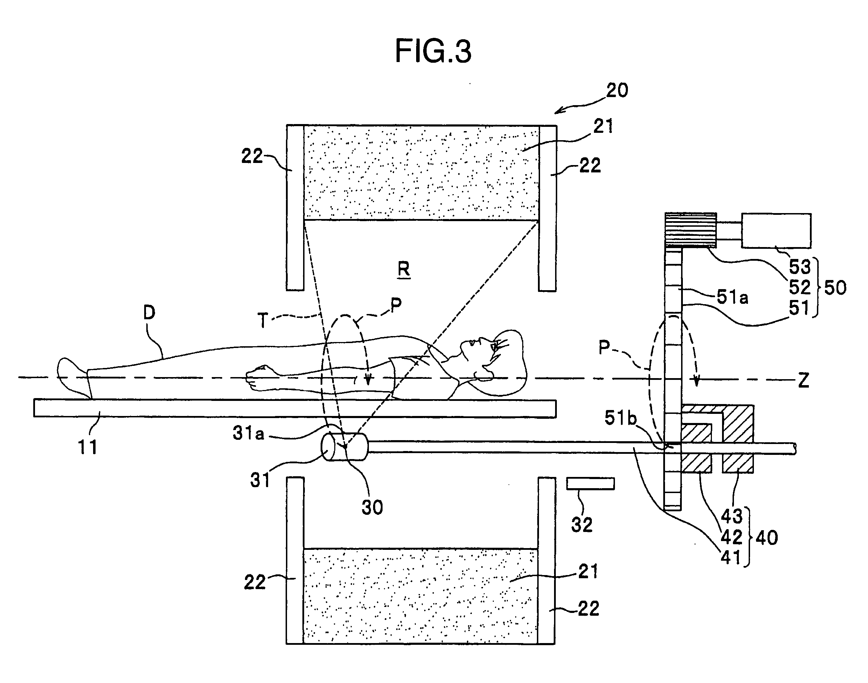

An explanation will be continued with reference to Fig. 3.

The imaging unit 20 comprises the radiation detector units 21, end shields 22, a correction radiation source 30, a radiation source holding unit 31, a shielding member 32, support means 40, and revolving means 50.

With the imaging unit 20 constructed in this manner, the test object D loaded on the bed 11 is inserted into the measurement space R, γ-rays (radiation) radiated from within a body of the test object D are detected in the imaging process, and the imaging data required for formation of a tomographic image are output to the data processing unit 12 (see Fig. 1).

Also, in the correcting process, the imaging unit 20 outputs to the data processing unit 12 (see Fig. 1) the imaging data required for recovery of that decrease in image quality, of the tomographic image, which is generated by decay caused when the γ-rays penetrate through the test object D.

The radiation detector units 21 are constructed so that a cylindrical-shaped hollow region, which a plurality of the detector units are arranged circumferentially to define, makes the measurement space R. The multiplicity of radiation detectors (for example, several tens of thousands to several hundreds of thousands) of the radiation detector units 21 are arranged densely not only on a plane in contact with the measurement space R but also along a depth of the radiation detector units.

In the imaging process, the radiation detector units 21 constructed in this manner detect those positions on the outer peripheral surface of the measurement space R, on which a pair of γ-rays radiated in mutually opposite directions (180 degrees ± 0.6 degrees) from within a body of the test object are incident, with high accuracy. Further, in the correcting process, the radiation detector units 21 detect crest values of the radiation T for correction, penetrating through the test object D in order to know the decay characteristic of y-rays in the test object D.

The end shields 22 are made of a lead material having an excellent shielding property for γ-rays and provided in pair on both cylindrical-shaped ends, at which the radiation detector units 21 are arranged circumferentially.

The end shields 22 constructed in this manner provide for shielding so that γ-rays do not enter the radiation detector units 21 from outside and the radiation T for correction from the correction radiation source 30 does not leak outside.

The correction radiation source 30 comprises, for example, germanium-68-gallium-68 (⁶⁸Ge-⁶⁸Ga) of 511 KeV in energy and cesium-137 (¹³⁷Cs) of 662 KeV in energy.

The correction radiation source 30 constructed in this manner radiates the radiation T for correction toward the test object D to clarify the decay characteristic when γ-rays detected by the radiation detector units 21 penetrate through the test object D.

The reason why it is necessary to use the correction radiation source 30 in this manner to examine the decay characteristic of γ-rays is that γ-rays radiated from a surface portion of the test object D and γ-rays radiated from a deep layer portion thereof are different from each other in detection efficiency of γ-rays detected by the radiation detector units 21. This is because γ-rays radiated from within a body of the test object D are absorbed and scattered to decay in the course of advancing in the body. Such decay of radiation degrades a tomographic image of the test object as imaged in image quality.

Here, that correction data, which are obtained by using the radiation detector units 21 to detect the radiation T for correction, penetrating through the test object D in the correcting process, are applied to that imaging data, which are obtained by using the radiation detector units 21 to detect γ-rays radiated from the test object D in the imaging process, thus improving a tomographic image in image quality.

The radiation source holding unit 31 is made of a lead or tungsten material to accommodate therein the correction radiation source 30. The radiation source holding unit 31 is fixed to a tip end of a support rod 41 described later so that an opening 31a communicated outside from that portion, in which the correction radiation source 30 is accommodated, is directed along a central axis Z of the measurement space R.

The radiation source holding unit 31 constructed in this manner serves to hold the correction radiation source 30 to set a direction, in which the radiation T for correction as output advances, to a predetermined direction and to inhibit radiation from leaking in other directions than the predetermined direction.

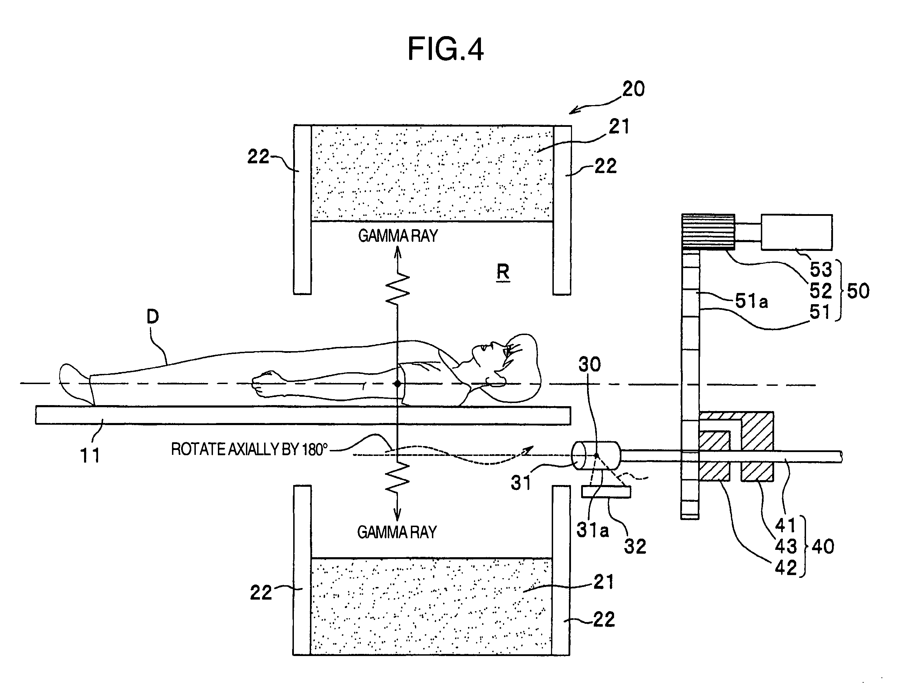

The shielding member 32 is arranged in the vicinity of the opening 31a of the radiation source holding unit 31, which is withdrawn from the measurement space R, to shield the radiation T for correction, radiated from the radiation source holding unit 31 (appropriately, see Fig. 4).

The support means 40 further comprises the support rod 41, an axial rotation angle imparting portion 42, and a linear displacement imparting portion 43.

The support means 40 constructed in this manner displaces the radiation source holding unit 31 along a body axis Z of the test object D and supports the radiation source holding unit so as to enable switching an advancing direction of the radiation T for correction. In the imaging process, the support means 40 withdraws the radiation source holding unit 31 outside from the measurement space R so that the radiation T for correction is not radiated on the test object D and the radiation detector units 21. Also, in the correcting process, the support means 40 causes the radiation T for correction, radiated from the radiation source holding unit 31 arranged within the measurement space R to penetrate through the test object D to be incident upon the radiation detector units 21.

The support rod 41 is in the form of a lengthy rod arranged along the body axis Z of the test object D and fixes to its tip end the radiation source holding unit 31. A base end of the support rod 41 is fitted into a hole 51b of a revolving rotator 51 described later to enable displacement in a longitudinal direction and axial rotation.

The axial rotation angle imparting portion 42 is structured so as to have the support rod 41 extending therethrough and through the hole 51b to guide the support rod 41 in a manner to enable the same to be displaced in a longitudinal direction and to impart an axial rotation angle to the support rod 41.

The axial rotation angle imparting portion 42 structured in this manner enables axial rotation of the support rod 41 to vary an orientation of the opening 31a inside and outside the measurement space R to optionally switch the advancing direction of the radiation T for correction.

As an example of the axial rotation angle imparting portion 42, a construction is conceivable, in which key slots are provided on a hollow rotor, which rotates relative to a stator fixed to the revolving means 50, in a manner to permit the support rod 41 to be displaced only in the longitudinal direction, thus having the support rod 41 fitted thereinto.

Also, as another example of the axial rotation angle imparting portion 42, a construction is also conceivable, in which twisted slots being not shown are provided on sliding surfaces of the support rod 41 and the axial rotation angle imparting portion 42 to fit mutually and an axial rotation angle is varied in proportion to a displacement of the support rod 41 in the longitudinal direction.

The linear displacement imparting portion 43 is structured to have the support rod 41 extending therethrough and through the hole 51b and the axial rotation angle imparting portion 42, to guide the support rod 41 in a manner to enable axial rotation of the same, and to impart longitudinal displacement to the support rod 41.

The linear displacement imparting portion 43 structured in this manner causes longitudinal displacement of the support rod 41 to move the radiation source holding unit 31 inside and outside the measurement space R (appropriately, see Fig. 4).

As an example of the linear displacement imparting portion 43, a construction is conceivable, which is fixed at one end thereof to the revolving means 50 described later and has the other end thereof supporting a distal end of the support rod 41 to enable axial rotation of the same, and which is expanded and contracted in a direction along a rotational axis Z by an action of hydraulic pressure or air pressure.

Also, as another example of the linear displacement imparting portion 43, a construction is conceivable, in which a rotating roller is provided on that slide surface of the axial rotation angle imparting portion 42, on which the support rod 41 slides, the rotating roller is brought into contact with the support rod 41 when the support rod 41 should be fed in the longitudinal direction, and contact of the rotating roller is released when axial rotation of the support rod 41 should be made.

The revolving means 50 further comprises the revolving rotator 51, a pinion 52, and a motor 53.

The revolving means 50 constructed in this manner revolves the support means 40 about the body axis Z of the test object D, thus causing the radiation source holding unit 31 arranged in the measurement space R to revolve about the body axis Z of the test object D. The revolving means 50 operates in this manner whereby the radiation T for correction is radiated omnidirectionally on the test object D and the penetrating radiation T for correction is incident upon the radiation detector unit 21, which is positioned in symmetry to the correction radiation source 30 with respect to the body axis Z. In this manner, the correcting process is executed.

The pinion 52 meshing with a gear 51a provided on an outer periphery of the revolving rotator 51 is driven and rotated by the motor 53 whereby the revolving rotator revolves round the rotational axis Z, which agrees with the central axis Z of the measurement space R. The support means 40 is provided on a surface of the revolving rotator 51 close to the outer periphery thereof to support the radiation source holding unit 31.

The revolving rotator 51 constructed in this manner revolves the support means 40 in a revolving track P when the motor 53 is driven. The radiation source holding unit 31 supported corresponding to the revolving rotator also revolves in the revolving track P about the body axis Z.

### (Description of operation)

Subsequently, an operation of the nuclear medicine diagnostic apparatus according to the embodiment of the invention will be described with reference to Figs. 3 and 4. Prior to explaining the imaging process, in which a tomographic image of the test object D is imaged, an explanation will be given to the correcting process, in which correction data required for an improvement in image quality, of the tomographic image are obtained.

In addition, it is assumed in a process of an actual PET diagnosis that either of the following alternatives is possible with respect to which of the imaging process and the correcting process should be executed and whether the correcting process should be executed before or after the test object D is given a radioactive medicine.

First, the bed 11 loading thereon the test object D in the correcting process is inserted into the measurement space R. The support means 40 is driven to arrange the radiation source holding unit 31 inside the measurement space R as shown in Fig. 3 from a withdrawal position outside the measurement space R. Further, the support means 40 operates so that the advancing direction of the radiation T for correction is directed toward the test object D, that is, the opening 31a is directed toward the body axis Z.

Subsequently, the revolving means 50 is operated so that the correction radiation source 30 goes round the body axis Z of the test object D with the opening 31a directed toward the test object D. Thereby, the radiation T for correction is radiated omnidirectionally on the test object D. The radiation T for correction, as omnidirectionally radiated, penetrates through the test object D to be detected by the radiation detector units 21, so that correction data are obtained to correct the decay characteristic of γ-rays in the test object D.

In addition, provided that the radiation T for correction is scattered as indicated by broken lines in Fig. 3 and correction data are acquired in a three-dimensional manner, the revolving means 50 suffices to rotate through one revolution, but provided that the radiation T for correction is not scattered and correction data are acquired in a two-dimensional manner, the correction radiation source 30 is required to rotate through several revolutions while being displaced along the body axis Z. Alternatively, the bed 11 loading thereon the test object D may be displaced along the body axis Z while a position of the correction radiation source 30 in the direction along the body axis Z is remained intact.

Subsequently, an operation until shifting to the imaging process after termination of the correcting process will be described with reference to Fig. 4.

First, after an operation of the revolving means 50 is stopped, the support means 40 is operated to withdraw the correction radiation source 30 outside the measurement space R from inside. When the correction radiation source 30 is positioned about the shielding member 32 arranged outside the measurement space R, the support rod 41 is caused to make axial rotation. Then, the opening 31a is switched in orientation, so that the advancing direction of the radiation T for correction is reversed. Thereby, the radiation T for correction advances in a reverse direction to the test object D and the radiation detector units 21, and is shielded by the end shields 22, the radiation source holding unit 31 itself, and the shielding member 32, so that the radiation T for correction is not incident upon the radiation detector units 21.

By the way, a procedure, in which the radiation source holding unit 31 positioned in the measurement space R is caused to make axial rotation of 180° to withdraw to a position about the shielding member 32, can adopt any one of the case where the radiation source holding unit 31 remained stationary inside the measurement space R is caused to make axial rotation of 180° and the support rod 41 is displaced in the longitudinal direction to withdraw to a position about the shielding member 32, the case where the radiation source holding unit 31 is caused to make axial rotation of 180° to withdraw to a position about the shielding member 32 while the support rod 41 is displaced, and the case where the radiation source holding unit 31 is caused to withdraw to a position about the shielding member 32 and then caused to make axial rotation of 180°, and so on.

In this manner, when the radiation T for correction reaches a withdrawal position outside the measurement space R, the procedure proceeds continuously to the imaging process. In the imaging process, the radiation detector units 21 counts γ-rays emitted from the test object D whereby imaging data required for obtaining a tomographic image of the test object are acquired. In the imaging process, the radiation T for correction, emitted from the correction radiation source 30, is not erroneously counted (contamination) by the radiation detectors, so that a tomographic image of the test object being high in image quality is obtained.

### (Second embodiment)

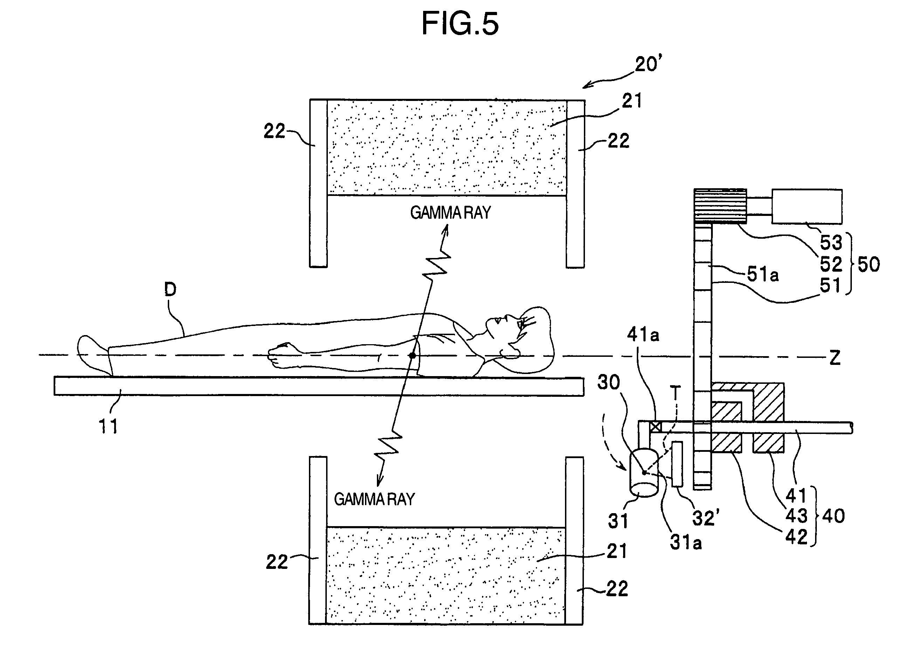

Subsequently, a PET apparatus, according to a second embodiment, preferred as a nuclear medicine diagnostic apparatus of the invention will be described with reference to Fig. 5.

An imaging unit 20' applied to the PET apparatus according to the embodiment further comprises articulation means 41a, which bends a support rod 41, as shown in Fig. 5. According to the second embodiment, a position, in which a shielding member 32' is arranged, is modified in conjunction with that construction, in which such articulation means 41a is provided. In addition, those remaining constituents, which constitute the imaging unit 20', are denoted by the same reference numerals as those described above and an explanation therefor is omitted.

The articulation means 41a is structured to bend a tip end of the support rod 41, to which a radiation source holding unit 31 is fixed, to switch an advancing direction of radiation T for correction.

As shown in Fig. 5, the articulation means 41a is set so that a direction, in which the support rod 41 is bent, substantially agrees with a diametrical direction of a revolving track P (see Fig. 3) of the articulation means 41a. In a state, in which displacement of the support rod 41 in a longitudinal direction is appropriately adjusted and the radiation source holding unit 31 is withdrawn outside a measurement space R, the support rod 41 is caused to make axial rotation of 180° and then the articulation means 41a is bent 90° radially outward whereby an advancing direction of the radiation T for correction is directed substantially in opposition to a test object D as shown in the figure. Thereby, the radiation T for correction, radiated from the correction radiation source 30 through an opening 31a, is radiated in an opposite direction to the test object D and radiation detector units 21.

Also, while depiction is omitted, a direction, in which the support rod 41 is bent by the articulation means 41a, may be set so as to agree substantially with a tangential direction (that is, a direction perpendicular to the plane of the figure) of the revolving track P (see Fig. 3) of the articulation means 41a. In this case, in a state, in which the radiation source holding unit 31 is withdrawn outside the measurement space R, the support rod 41 is caused to make axial rotation of 90° and then the articulation means 41a is bent 90° in the tangential direction whereby an advancing direction of the radiation T for correction is directed substantially in opposition to the test object D. Thereby, the radiation T for correction, radiated from the correction radiation source 30 through the opening 31a, is radiated in the opposite direction to the test object D and radiation detector units 21.

In this case, since a displacement magnitude of the support rod 41 can be decreased when the radiation source holding unit 31 is withdrawn outside the measurement space R, a dimension of the PET apparatus in a direction along a body axis can be decreased.

As described above, with the nuclear medicine diagnostic apparatus according to the invention, after the correcting process is terminated, the radiation source holding unit 31 is withdrawn outside the measurement space R in a state, in which it is caused to make axial rotation and the support rod 41 supporting the radiation source holding unit 31 at its tip end is bent 90°. Thereby, an advancing direction of the radiation T for correction, radiated from the correction radiation source 30, is directed in opposition to the radiation detector units 21, so that it is possible to completely prevent contamination, in which the radiation T for correction, radiated from the correction radiation source 30, is erroneously counted by the radiation detector units 21, in the imaging process.

Further, there is no need of providing an exclusive moving shielding vessel, which accommodates therein the correction radiation source 30 while shielding it, and ensuring a large distance between the correction radiation source 30 as withdrawn, and the test object D and radiation detector units 21, so that the PET apparatus 10 is not made large-scale and space occupancy in a medicine diagnostic facility is reduced, thus enabling achieving space saving. Also, an operation in shielding is made simple and stable.

While the embodiments of a PET apparatus have been described as a nuclear medicine diagnostic apparatus of the invention, the nuclear medicine diagnostic apparatus of the invention is not limited to a PET apparatus but applicable to a general nuclear medicine diagnostic apparatus provided with a correction radiation source. The nuclear medicine diagnostic apparatus is applicable to, for example, a SPECT apparatus.

It should be further understood by those skilled in the art that although the foregoing description has been made on embodiments of the invention, the invention is not limited thereto and various changes and modifications may be made without departing from the spirit of the invention and the scope of the appended claims.

## Claims

1. An nuclear medicine diagnostic apparatus comprising
a radiation detector (21) that detects radiation radiated from a test object (D),
a correction radiation source (30) used to correct decay generated when the radiation radiated by the radiation detector (21) penetrates through the test object,
a radiation source holding unit (31) that holds the correction radiation source (30) to set an advancing direction of radiation for correction as output to a predetermined direction,
support means (40) that displaces the radiation source holding unit (31) along a body axis (Z) of the test object (D) and supports the radiation source holding unit (31) in a manner to enable switching the advancing direction of the radiation for correction, and
revolving means (50) that revolves the support means (40) about the body axis (Z).

2. The nuclear medicine diagnostic apparatus according to claim 1, **characterized in that** the support means (40) comprises
a support rod (41), to which the radiation source holding unit is fixed and which is arranged along the body axis (Z) of the test object,
an axial rotation angle imparting portion (42) that imparts an axial rotation angle to the support rod (41), and
a linear displacement imparting portion (43) that imparts a lengthwise displacement to the support rod (41).

3. The nuclear medicine diagnostic apparatus according to claim 2, **characterized in that** further comprises articulation means (41a) that bends the support rod (41).

4. The nuclear medicine diagnostic apparatus according to claim 3, **characterized in that** a direction, in which the support rod (41) is bent by the articulation means (41a), substantially agrees with a diametrical direction of a revolving track of the articulation means (41a) and the axial rotation angle and the lengthwise displacement are imparted to the support rod (41) so that the advancing direction of the radiation for correction is directed substantially in opposition to the test object (D).

5. The nuclear medicine diagnostic apparatus according to claim 3, **characterized in that** a direction, in which the support rod (41) is bent by the articulation means (41a), substantially agrees with a tangential direction of a revolving track of the articulation means (41a) and the axial rotation angle and the lengthwise displacement are imparted to the support rod (41) so that the advancing direction of the radiation for correction is directed substantially in opposition to the test object (D).

6. The nuclear medicine diagnostic apparatus according to claim 1, **characterized in that** it further comprises a shielding member (32, 32') that shields the radiation for correction, output from the radiation source holding unit (31).

7. The nuclear medicine diagnostic apparatus, according to claim 1, **characterized in that** the apparatus is applied to positron emission computed tomography.
